(19) Europäisches Patentamt
European Patent Office
Office européen des brevets

(11) **EP 3 504 649 B1**

(12) **EUROPEAN PATENT SPECIFICATION**

(45) Date of publication and mention
of the grant of the patent:
**13.05.2020 Bulletin 2020/20**

(51) Int Cl.:
***G16H 40/63*** *(2018.01)*   ***G16H 40/67*** *(2018.01)*
***G16H 50/30*** *(2018.01)*

(21) Application number: **17761458.3**

(22) Date of filing: **24.08.2017**

(86) International application number:
**PCT/EP2017/071320**

(87) International publication number:
**WO 2018/037073 (01.03.2018 Gazette 2018/09)**

(54) **DEVICE, SYSTEM AND METHOD FOR PATIENT MONITORING TO PREDICT AND PREVENT BED FALLS**

VORRICHTUNG, SYSTEM UND VERFAHREN ZUR PATIENTENÜBERWACHUNG ZUR VORHERSAGE UND VERHINDERUNG VON BETTSTÜRZEN

DISPOSITIF, SYSTÈME ET PROCÉDÉ DE SURVEILLANCE D'UN PATIENT POUR PRÉDIRE ET PRÉVENIR LES CHUTES DE LIT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.08.2016 EP 16185507**

(43) Date of publication of application:
**03.07.2019 Bulletin 2019/27**

(73) Proprietor: **Koninklijke Philips N.V.**
**5656 AG Eindhoven (NL)**

(72) Inventor: **WEFFERS-ALBU, Mirela, Alina**
**5656 AE Eindhoven (NL)**

(74) Representative: **Kroeze, Johannes Antonius**
**Philips Intellectual Property & Standards**
**High Tech Campus 5**
**5656 AE Eindhoven (NL)**

(56) References cited:
**WO-A1-2015/074007**

• **CHARALAMPOS N DOUKAS ET AL: "Emergency Fall Incidents Detection in Assisted Living Environments Utilizing Motion, Sound, and Visual Perceptual Components", IEEE TRANSACTIONS ON INFORMATION TECHNOLOGY IN BIOMEDICINE, IEEE SERVICE CENTER, LOS ALAMITOS, CA, US, vol. 15, no. 2, 1 March 2011 (2011-03-01), pages 277-289, XP011373669, ISSN: 1089-7771, DOI: 10.1109/TITB.2010.2091140**

**Description**

FIELD OF THE INVENTION

The present invention relates to a device for monitoring individuals, especially patients in a hospital or under homecare, the device being adapted to allow prediction of an eminent fall of the person out of bed. The device emits an alarm when a number of connected risk factors exceed a defined threshold. The present invention further relates to a system incorporating the aforementioned device and a method of monitoring individuals to predict bed falls.

BACKGROUND OF THE INVENTION

Falls are the most common adverse event reported in hospitals and are a leading cause of hospital-acquired injury, and frequently prolong or complicate hospital stays. Reviews of observational studies in acute care hospitals show that fall rates range from 1.3 to 8.9 falls/1,000 patient days and that higher rates occur in units that focus on eldercare, neurology and rehabilitation. In spite of extensive research on falls risk factors and the development of a number of falls risk instruments, protocols are applied inconsistently, and risk factor directed interventions are far from standardized.

Although falls can occur at all ages, they are known to lead to significant injury in older people or those from high falls risk populations when compounded by an acute health problem requiring hospitalization, or for those requiring admission to residential care settings.

In addition, other observational studies show that 60-70% of all falls in hospital occur from the bed or bedside chair, that more than 80% of falls are unwitnessed and that about 50% occur in patients who fall repeatedly.

Given the high impact of fall incidents on patients' health and quality of life as well as costs of care, finding scalable and cost efficient solutions for patient monitoring and fall prevention to reduce the number of bed falls incidents becomes of utmost importance.

The current state of art includes solutions such as sitter services as means for bed fall incidents prevention, bed rails as means for bed fall incidents prevention and certain automatic solutions for patient monitoring.

Sitter service is difficult to implement, not scalable and not cost efficient. Towards its implementation hospitals typically must choose between employing sitters from outside the hospital staff which is financially taxing due to the service high costs or assigning sitter duties to their own hospital staff, which increases significantly the responsibilities and workload of the staff, typically already overburdened due to staff shortages. In addition assigning sitter tasks (that do not require medical training) to qualified staff prevents appropriate use of personnel qualifications and skills.

In that sense sitter services do not present a favorable outlook when it comes to implementing a reliable, scalable and cost efficient prevention strategy for inpatient bed fall incidents.

Bed rails as a single prevention strategy do not seem to guarantee the prevention of bed fall incidents. 50-90% of falls from bed in hospital occur despite bedrails being applied, showing limited success in preventing falls in general. In addition bedrail use may also be associated with worsening of agitation, fear and delirium. "Chemical" restraint e.g. in the form of neuroleptic use despite the misguided intention to prevent falls by its use is associated with increased fall rates. Moreover, restraint or bed rail use can lead to muscle wasting, infection or pressure sores from immobility, and deconditioning. Finally there is an ethical component to be considered when it comes to restricting patient moving ability.

Technology based products have been used in an attempt replace sitter service but may also have innate drawbacks. Some systems do not provide any intelligence to support the so-called "eSitter" in monitoring patients, which limits the number of patients monitored in parallel. As the scalability of such solutions is limited, up-scaling requires additional devices and remote monitoring stations. No intelligence implemented to determine automatically the fall risk in real time raises concerns and puts in question the feasibility of fall incidents prevention. Fall risk assessed is based on input obtained upon initial and ongoing patient contact by hospital staff but is not updated based on continuous automatic observation of patient in real time. As updates of the fall risk are based on interviews with the patient at admission and during hospitalization, these updates are liable to inaccuracy and significant delays, raising further concerns.

Other systems implement a technical approach where whenever a partial bed edge crossing happens (by the blanket or by patient arm) without an actual patient bed-exit, the system triggers an alarm. This leads to a high rate of false alarms.

In addition regarding effective preventive interventions, these technologies focus very much on monitoring and alarms but no attention is given to understanding the most optimal intervention that should be provided given a particular patient profile at a certain time. Furthermore, if medical staff should intervene, these systems do not provide support in determining the actual persons who should be notified, in order to optimize staff resources. Finally all alerts are issued only when the risk of falling is very high and the incident occurrence is imminent, at which point the chance of providing an effective intervention that actually prevents the fall is decreased.

WO 2013/150523 A1describes monitoring, prediction and treatment of clinical episodes. Apparatus and methods are provided for use with a patient who has been assigned a treatment protocol. A sensor senses at least one physiological parameter of the patient. A control unit includes signal analysis functionality that analyzes the physiological parameter and determines whether the patient has followed the treatment protocol. A user interface generates an output to the patient that is indicative of (a) a correlation between the physiological parameter and non-compliance by the patient with the treatment protocol, the output being generated in response to the analysis functionality determining that the patient has failed to follow the treatment protocol, or (b) a correlation between the physiological parameter and compliance by the patient with the treatment protocol, the output being generated in response to the analysis functionality determining that the patient has followed the treatment protocol.

SUMMARY OF THE INVENTION

It is an object of the present invention to provide a device, a system and a method for monitoring of patients which not only monitors the movement patterns and the vital signs of the patient but also assesses the data and evaluates a risk score which on the one hand allows reliable prediction and on the other hand minimizes false alarms, thus allowing efficient but attentive care for the patients.

In a first aspect of the present invention a device for adaptive intervention with an individual in connection with a bed fall risk of the individual is presented that comprises a first port for obtaining personal data of the individual and a total risk score of the individual, an intervention unit for determining a projection regarding an imminent bed fall of the individual and a timeframe for an intervention to prevent the bed fall of the individual based on the obtained data, and a second port for outputting an intervention signal based to the projection and the obtained data.

In a further aspect of the present invention a system for adaptive intervention with an individual in connection with a bed fall risk of the individual is presented that comprises a classification unit for providing personal data of the individual, a calculation unit for calculating a total risk score of the individual, and a device for adaptive intervention with the individual based on the output of the classification unit and the calculation unit.

In another aspect of the invention a method for determination of a bed fall risk of an individual is presented, the method comprising the steps of obtaining personal data of the individual, and obtaining a total risk score of the individual, determining a projection regarding an imminent bed fall of the individual and a timeframe for an intervention to prevent the bed fall of the individual based on the obtained data, and intervening at the individual based on the obtained data and the projection.

In yet a further aspect of the invention a computer program comprising program code means for causing a computer to carry out the steps of the method when said computer program is carried out on a computer is presented.

The inventive device, system, method and computer program for determination of a bed fall risk differ to the state of art described above in that an intervention unit is configured to on the one hand take into account the personal data of an individual and on the other hand detect risk scores and ability levels of the individual based on movement data and the health record and to decide based on the data collection how to intervene effectively and reliably at the individual to prevent a bed fall.

Preferred embodiments of the invention are defined in the dependent claims. It shall be understood that the claimed method has similar and/or identical preferred embodiments as the claimed device and as defined in the dependent claims.

Preferably the intervention unit is configured to output the intervention signal directly to the individual by way of an interface arranged in the vicinity of the individual. This is the easiest and most straight forward approach to intervene with the individual without referring to staff or other resources. If this direct intervention fails, the next step can be initiated.

According to an advantageous embodiment, the device further comprises a third port for obtaining data related to a staff schedule. If an alert is given out, the staff schedule can be taken into account and the staff which is nearest or is idle or has tasks of lower priority will be alerted first to assist the individual.

Advantageously the intervention unit is configured to output the intervention signal to a staff member and to trigger a staff intervention at the individual based on the staff schedule, the obtained data and the projection. Thus, the action will be streamlined with least risk for the person under care, reasonable effort of the staff and most effective intervention.

Preferably, the personal data of the individual comprises one or more of age, gender, neurologic condition, symptoms, disabilities, medication, fall history, psychological profile. These data are stored in any case in the care environment's IT system when the individual is accommodated and thus are easily available for determination of the risk score of the individual.

The total risk score advantageously is determined by obtaining the personal data of the individual and by obtaining sensor data from at least one sensor arranged in the vicinity of the individual and/or in contact with the individual. Thus an individual risk score based on both the actual behavior and the long-term health record of the individual can be customized, and the individual risk of a bed fall can be predicted with high accuracy.

According to a preferred embodiment of the invention the intervention unit is configured to distinguish distinct ability levels of the individual based on the total risk score and its change and/or development during a predetermined time-frame, wherein the ability levels of the individual comprise a cognitive ability level, a communication ability level, a compliance level. The different levels allow an easy and reliable assessment of the imminent risk of a bed fall and can be easily discerned from each other. Thus, the respective intervention can be triggered.

The cognitive ability level is related to the total risk score being low or moderate but slowly to moderately increasing within a limited time-window. This is the lowest level of intervention where the individual most probably can be attended to by the direct intervention by interface.

The communication ability level is related to the total risk score being low or moderate but significantly increasing within a limited time-window. In this case, the system will be alerted and ready to raise an alarm if the total risk score keeps increasing. If there is a decrease, the system will lower the alert status.

The compliance level is related to the total risk score being determined to be high. If the total risk score is permanently high, the system will be in permanent alert and keep the staff informed about the tight situation.

Preferably, the step of intervening at the individual comprises addressing the individual directly via an interface and/or triggering an intervention by staff based on a staff schedule, the obtained data and the projection. This allows to intervene effectively with the best use of all resources, thus streamlining the process and simultaneously allowing to monitor each person individually according to the respective needs.

BRIEF DESCRIPTION OF THE DRAWINGS

These and other aspects of the invention will be apparent from and elucidated with reference to the embodiment(s) described hereinafter. In the following drawings

 Fig. 1 shows a schematic overview over an inventive device and system for monitoring of patients and for predicting and preventing bed falls,
 Fig. 2 shows a schematic view of the bed frame covered by a video component,
 Figs. 3 to 10 show a patient in bed under different conditions,
 Figs. 11 to 13 show diagrams of accelerometer signals of certain patient's postures and movements,
 Fig. 14 shows a restlessness diagram of a patient, and
 Fig. 15 shows a diagram comparing restlessness and agitation of a patient.

DETAILED DESCRIPTION OF THE EMBODIMENTS

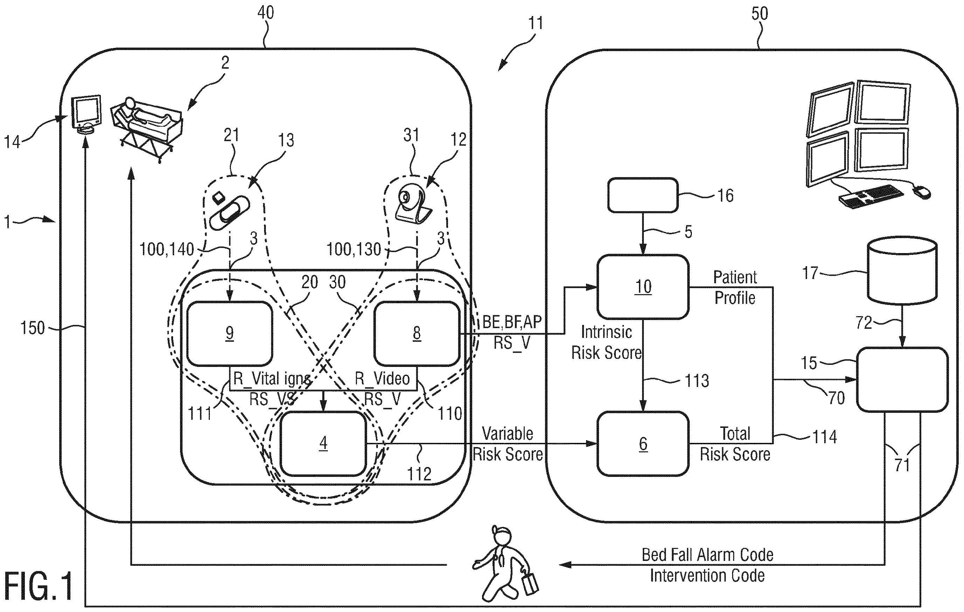

In Fig. 1, an overview of a preferred embodiment of the invention is diagrammatically shown. The embodiment comprises a device 1 for monitoring an individual 2. The individual 2 can be especially a patient in a hospital bed. In the following, the individual 2 thus will be addressed as patient 2, but the individual 2 could also be a resident of a nursing home, an occupant in a psychiatric ward, an individual 2 under home care or the like. The principle at the basis of the device 1 is that the bed fall risk associated with patients 2 under monitoring is determined both by unmodifiable risk factors such as age, certain debilitating (permanent) conditions, physical impairments etc. of the patient 2, as well as the psychologic make-up (regarding level of compliance/adherence to medical guidelines in the hospital) and modifiable risk factors such as restlessness, level of confusion, level of anxiety, type and speed of movement (e.g. erratic movements) of the patient 2 while occupying the bed.

The device 1 ensures that the calculation of a risk score associated with each patient 2 is based on an assessment of all risk factors above (unmodifiable and modifiable), and that intervention strategies implemented are tailored to each patient 2 to ensure full effectiveness of the solution by preventing bed fall incidents as well as optimization of medical staff resources by only involving the medical personnel when needed, and then involving the staff most accessible and/or available to accomplish the intervention.

The device 1 of the embodiment of Fig. 1 connects via first ports 3 to sensors 12 and 13 being located in a patient room 40 where the patient 2 is situated. The embodiment especially provides the sensor 12 in form of a video camera 12 or another suitable device for monitoring movement of the patient 2 and the sensor 13 in form of a vital sign sensor 13. The vital sign sensor 13 can in particular be a photoplethysmography sensor, a heart rate monitor, a blood pressure monitor, an SpO2 sensor and/or a respiration monitor which all are suitable to evaluate vital signs and simultaneously allow conclusions to be drawn about stress, anxiety, agitation and the like. The vital sign sensor 13 can be arranged on the patient's body or in a remote location, especially in case when a PPG sensor is used. The sensors 12, 13 taking input in real time either continuously or at discrete intervals thus provide a certain redundancy for the monitoring, allowing an accurate and failsafe prediction of incidents. In case one of the sensors 12, 13 is out of order, the other still can provide data.

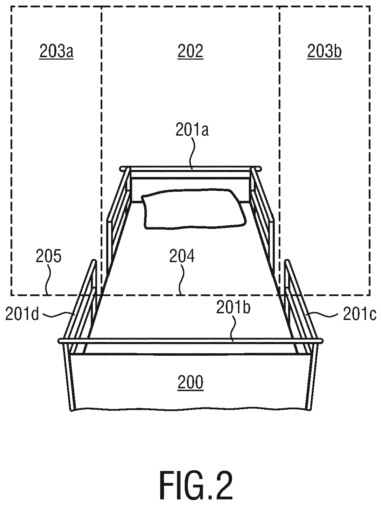

Sensor data 100 collected by the sensors 12, 13, i.e. video data 130 and vital signs data 140, are transferred to a video processing unit 8 and a vital signs processing unit 9, respectively. The processing units 8, 9 are configured to detect in real time risk factors by analyzing the video data 130 and the vital signs data 140 and to calculate a risk score 110, 111 for each data set based on the risk factors detected. The video processing unit 8 and the vital sign processing unit 9 and their functions will be described later in more detail with reference to Figs 2 to 15.

The device 1 further contains an evaluation unit 4 for determining quality information indicating the quality of the sensor data 100. Thus the reliability of the video data 130 and the vital signs data 140 are assessed and weighted. The evaluation unit 4 obtains the risk scores 110, 111 from the respective processing units 8, 9, determines a reliability value for the respective data 130, 140 and calculates a variable risk score 112 therefrom as described later in more detail. The vital signs processing unit 9 in connection with the evaluation unit 4 may be addressed as vital signs risk score detection device 20, whereas the video processing unit 8 in connection to the evaluation unit 4 may be addressed as video data risk score detection device 30. The respective video sensor 12 and vital sign sensor 13 in connection with the respective devices 30 and 20 may be addressed as systems 31 and 21.

The evaluation unit 4 uses the risk factors 110 and 111 (defined above) as well as sensor reliability indicators (as described in detail further below) in order to calculate in real-time an overall patient variable risk score 112 for bed fall incidents. The variable risk score 112 is transmitted to a calculation unit 6 for further processing. Especially, the variable risk score 112 can be transmitted from the patient room 40 to a remote workstation room 50 via a network communication protocol.

The sensor reliability indicates the level of confidence associated with each sensor depending on environmental and connectivity factors as described in detail with reference to the functionality of the video and vital signs processing units 8, 9 further below.

As such the variable risk score 112 is defined to be a composite of the R_Video and R_ VitalSigns (defined above) where the sensors reliability indicators (SR_V and SR_VS) act as weighting factors. As an example, in a potential implementation if both SR_V and SR_VS are 0, then the Variable Risk Score is set to -1, indicating the system is not able to calculate the Variable Risk Score due to unreliable input. In all other cases:

$$\text{Variable Risk Score} = (SR\_V * R\_Video + SR\_VS * R\_VitalSigns) / (SR\_V + SR\_VS)$$

The formula above implies that whenever the video signal is not reliable, the variable risk score 112 is in effect equal to R_ VitalSigns. Conversely, if the vital signs signal is not reliable, the variable risk score 112 is in effect equal to R_Video. If both signals are viable then the variable risk score 112 is an average of the two risk scores 110 and 111 above. Expressed in pseudocode:

```
if (SR_V =0 AND SR_VS = 0) then
         variable risk score = -1
         else
         variable risk score = (SR_V* R_Video + SR_VS* R_VitalSigns) / (SR_V +
         SR_VS).
```

The components previously described are arranged in the vicinity of the patient 2. The following components however advantageously are arranged in the remote workstation room 50.

A classification unit 10 classifies patients 2 based on their clinical and psychological profiles and provides an intrinsic risk score 113 based on the patient profile. The patient profile in the current embodiment is obtained from a database 16 via a second port 5. The patient profile includes any information available about the patient 2, starting with age and gender and further containing the current diagnosis, respective medication, the allover condition of the patient, the medical history and the bed fall history and so on. The patient profile thus is a major source for assessment of the bed fall risk of a patient 2.

The personal data 120 provide unmodifiable risk factors constituting the clinical and psychological profiles, and provide the intrinsic risk score 113 therefrom.

Unmodifiable risk factors may be patient age (older patients are at higher risk of developing cognitive impairments and therefore would have an increased bed fall risk), patient gender (male patients appear to be at higher risk for falling incidents, possibly due to higher reluctance to receive assistance when leaving the bed), (neurologic) condition (dementia (e.g. Alzheimer) patients and patients with Parkinson's Disease are known to run a higher risk of falling out of bed due to being more prone to spatial disorientation, agitation episodes, and acting out their dreams due to sleeping disorders. Having a neurological disease would correspond to increased bed fall risk level as opposed to not having these diseases), current or previous symptoms (registered/observed occurrences of hallucination / sleeping disorders will increase the bed fall risk levels, (cognitive, visual) disabilities (visually impaired patients will run a higher risk of not estimating well distances, be spatially unaware contributing to an increased bed fall risk level), medications (large number of prescription medications, or patients taking medicines that could cause sedation, confusion, impaired balance, or orthostatic blood pressure changes are at higher risk for falls, bed falling history (consistent history of bed falling episodes will increase the likelihood of a subsequent repeated event as opposed to sporadic (or no previous history) of such events), and psychologic profile - compliance / adherence level (patients with lower levels of compliance / adherence to medical guidelines during their hospitalization (e.g. do not leave the bed un-attended) are at higher risk for falling while attempting to exit the bed than otherwise).

All unmodifiable factors mentioned above (except bed fall history and psychological profile) are determined by the classification unit 10 from the patient electronic health record 16 as shown in Fig. 1.

Patient bed fall history is assessed via a simple question regarding the number of falls the patient experienced so far. Patient psychological profile (regarding the patient level of compliance / adherence to medical guidelines in the hospital) is determined by means of a questionnaire test at hospitalization as a way of determining a baseline. This profile is updated based on further learning regarding the patient level of compliance/adherence with medical advice to not leave the bed un-attended, during the hospital stay.

The intrinsic risk score 113 is based on assessing the unmodifiable risk factors mentioned above, where the intrinsic risk score is increased by a parameter $x_i$ (i=1..8) - associated with each risk factor. This would imply the following tests and associated computations:

If Patient Age > 65 then
Intrinsic Risk Score = Intrinsic Risk Score $\otimes$ x1
If Patient Gender = male then
Intrinsic Risk Score = Intrinsic Risk Score $\otimes$ x2
If Patient (Neurologic) condition = yes then
Intrinsic Risk Score = Intrinsic Risk Score $\otimes$ x3
If Patient disabilities (visual or motoric) = yes then
Intrinsic Risk Score = Intrinsic Risk Score $\otimes$ x4
If Patient medication (causing sedation etc.) = yes then
Intrinsic Risk Score = Intrinsic Risk Score $\otimes$ x5
If Patient symptoms = yes then
Intrinsic Risk Score = Intrinsic Risk Score $\otimes$ x6
If Patient having a bed falling history = yes then
Intrinsic Risk Score = Intrinsic Risk Score $\otimes$ x7
If Patient level of adherence = low then
Intrinsic Risk Score = Intrinsic Risk Score $\otimes$ x8
wherein in a simple example "$\otimes$" could stand for a simple sum.

At system initialization parameters x1 to x8 can be initialized based on values indicated by literature studies, while over time, the system adjusts the parameters values by learning the influence of each risk factor above on a bed fall occurrence, for the specific population at hand. This is done by executing correlation analyses and applying regression techniques after each bed fall incident to understand the impact of each risk factor on the likelihood of a bed fall event and to update the values of the parameters above. All risk factors indicated above are known from medical literature to have a causative relevance to the occurrence of fall incidents. The question remains how much of a causative relevance each risk factor has for each patient (given their certain patient profile). Parameters x1 to x8 quantify the causative relevance of their associated risk factors, this making their determination necessary. Determination is done by evaluation of the level of correlation between the presence of their (associated) risk factors and the occurrence of a fall incident. That is, the higher the number of times the occurrence of a risk factor precedes the occurrence of a fall incident, the higher the likelihood that the risk factor has a higher causative relevance to the occurrence of a fall incident for a patient with a certain profile. If a risk factor causes a fall incident, then it has a predictive power as well. Correlation quantifies the degree to which two variables are related - in this case a risk factor and a fall occurrence. Computing a correlation coefficient (r) indicates how much one variable tends to change when the other one does. For that reason in an embodiment, parameters x1 to x8 could be represented by the correlation coefficients (or a factor of these) for example. As time passes the systems becomes increasingly accurate in determining this intrinsic risk. If no bed fall incident occurs no parameter adjustment is needed.

Further, a calculation unit 6 is provided which obtains the variable risk score 112 from the evaluation unit 4 and the intrinsic risk score 113 from the classification unit 10 and therefrom calculates a total risk score 114. The latter is together with the patient profile obtained from the database 16 the input to an intervention unit 15 which provides strategies for the prevention of an imminent bed fall of a patient 2 based on the patient's clinical and psychological profile and the total risk score 114. The total risk score 114 is a composite of the intrinsic risk score 113 and the variable risk score 112 values:

$$\text{Total Risk Score} = \text{Intrinsic Risk Score} \otimes \text{Variable Risk Score}$$

In a potential implementation the formula could express a weighted sum or weighted average of the two risk scores 112 and 113 above where the weights can be updated based on continuous system learning from the data of the population monitored expressing the level of impact of modifiable and unmodifiable risk factors.

The device 1 according to the preferred embodiment may further correspond to additional components like a feedback unit 14 which is located in the vicinity of the patient 2 and which can directly address the patient 2 in case the device 1 detects an imminent bed fall. Generally, the device 1 preferably has a configuration which provides as little components as possible in the patient room 40 and respectively the remote workstation room 50 where the other components are located. This configuration allows monitoring of a number of patients 2 in various locations without the necessity of a whole monitoring system for each patient 2.

According to the preferred embodiment of Fig. 1, in the patient room 40 only the following components are installed: the video camera 12, the vital signs sensor 13, the respective processing units 8, 9 and the evaluation unit 4. Alternatively, it would also be possible to arrange the processing units 8, 9 and the evaluation unit 4 in the remote workstation room 50 and to transmit the data 130, 140 e.g. by a wireless network thereto. Since nowadays modern video cameras 12 and vital signs sensors 13 are small components which can already be combined with their respective processing units 8, 9, these components can however be arranged unobtrusively in the patient room 40. Arrangement of the evaluation unit 4 in the vicinity of the processing units 8, 9 allows easier data administration since the individual sensor data 100 of each patient can be unambiguously assigned to the respective patient 2 and for example stored in a data storage (not shown) until they are requested from the calculation unit 6.

Further, the feedback unit 14 is arranged in the patient room 40. The feedback unit 14 can for example be a TV display equipped with a microphone which is normally provided anyway in each patient room 40 in a hospital for entertainment of the patients 2 and which can be used by the intervention unit 15 to communicate with the patient 2. Alternatively, the feedback unit 14 can be an additional, separate unit which can be part of a monitoring system which is rather used by the medical staff. In this case, the feedback unit 14 can also serve as a source of information for the staff entering the room in case there was an alert about an imminent bed fall.

In the following, the functionality of the video camera 12 and the respective processing unit 8 is described in more detail with reference to Figs. 2 to 10. For the sake of simplicity, in the following the video camera 12 and the assigned processing unit 8 are termed as "video component", referring to the components with the reference numbers 12 and 8.

As mentioned above, the video component implements two main functionalities: on the one hand, the reliability of the video data 130 is assessed, on the other hand, detection of the presence of risk factors in real time is carried out, based on which a video data risk score 110 indicating the likelihood of a bed fall incident in view of the parameters detected by the video camera 12 is calculated. This functionality is executed only if the reliability of the video data 130 mentioned above is determined to be sufficient.

Assessment of the reliability of the video data 130 can for example be done by use of a variable to express video sensor reliability (SR_V). SR_V is set on 1 when the sensor reliability is assessed to be sufficient and 0 otherwise.

The assessment is primarily based on illumination levels, an estimation of the contrast variations of the moving objects in the scene and the signal to noise ratio of the camera and consequently also of the video data 130. Signal in this application is the minimal contrast change in a scene as result of a movement of an object in the scene. In that sense whenever illumination levels or the signal to noise ratio of the video data 130 are below a threshold (e.g. 6 lux, 3dB signal to noise ratio), the sensor signal is determined to be not reliable and SR_V is set on 0. If both illumination and signal to noise ratio are above the threshold, SR_V is set on 1. The threshold depends on the camera type and quality used and must be set as such at initialization. This signal reliability assessment can be done either punctually with a certain frequency and/or during predetermined intervals or alternatively continually to check if the value of SR_V needs to be changed.

Detection of the risk factors and calculation of the video data risk score 110 is only done if the reliability value SR_V is found to be 1. For as long as the video data 130 are considered to be reliable, the video processing unit 8 processes the video data 130 and detects in real-time a number of risk factors which mainly refer to the patient's body posture and the position occupied in bed. Examples are: the patient's torso is up from a supine position, the patient 2 is restless, turns and tosses or the patient 2 is near the bed edge.

In addition the video component is able to detect events like intentional and unintentional bed exits of the patient 2. If the patient 2 exits the bed intentionally, a bed exit indicator will be set to 1 (BE=1) while otherwise it is set to 0. If the patient 2 has unintentionally fallen out of bed, a bed fall exit indicator will be set to 1 (BF=1) while otherwise it is set to 0. In case an additional person is present in room, an additional person indicator is set to 1 (AP=1) while otherwise it is set to 0.

Detecting the modifiable risk factors above allows calculating a video data risk score 110 for a bed fall incident based on the video data 130. If the camera is not reliable (SR_V = 0) the processing unit 8 does not process the signal at all and the video data risk score 110 is set to -1 to indicate that the risk has not been assessed due to signal unreliability. If the video signal is found not to be reliable, then the system will react based on the risk score from the vitals signs sensor 13 and vice-versa. If none of the signals are reliable then the device 1 sends a message to the intervention unit 15 that real-time monitoring is not possible and that a technical team needs to attend the patient room 40 to address issues and restore signal quality. According to a preferred embodiment, the device 1 can self-diagnose and write a log with the cause of the signal unreliability. In case of the vital signs signals this could e.g. be network bandwidth not sufficient, insufficient throughput, etc. In case of the video signal in-adequate illumination conditions etc. can be the cause of signal unreliability. The log can even provide statistics on the most present cause, location / patient room 40 most affected, etc. The log will then be used by the technical team to solve the issues.

The output of the video data processing unit 8 comprises the SR_V signal reliability indicator and the video data risk score 110. These data are delivered to the evaluation unit 4.

Besides, the video data processing unit 8 provides data for retrieval by the classification unit 10 to help learn the patient compliance levels, e.g. to not leave the bed unattended. The data handed over to the classification unit 10 are the SR_V signal reliability indicator, the BE and BF codes for detected intentional bed exit and unintentional bed fall events and the additional person indicator AP.

In the following, the algorithms for detection of the risk factors mentioned above are described. Reference is taken to Fig. 2 first.

In Fig. 2, a very schematic bed 200 for a patient 2 (not shown in Fig. 2) is illustrated.

The inventive device 1 for bed fall prediction assumes (x, y) coordinates of points indicating the bed space, e.g. the four bed edges 201a, 201b, 201c and 201d, in the frame covered by the video camera 12 to be known. The bed edges 201a, 201b, 201c and 201d can be indicated manually e.g. by drawing them in a visual editor which acts as an input device (not shown) or they could be detected automatically.

Based on the coordinates of the bed edges 201a, 201b, 201c and 201d, the algorithm executes the following steps:

1. Defines a first region of interest 202 and a second region of interest 203 which splits in two parts 203a and 203b arranged adjacent to the first region of interest 202 and adjacent of bed edges 201c and 201d, used later in detecting parameters;
2. Determines the valid motion trajectories within the second region of interest 203a and 203b, with valid trajectories being defined by a certain length and maximum variance determined based on learning;
3. Clusters valid motion trajectories to identify moving entities based on direction, slope, position and length over a number of image frames (currently 15 image frames - this threshold needs to be learned and adapted according to movement behavior in the scene, such as speed);
4. Determines the center of gravity motion of each entity by calculating the median of the x, y coordinates of the end point of all valid motion trajectories belonging to the entity;
5. Determines reference centers of motion gravity: the right-most center of motion gravity 401, this being the gravity center with the largest x coordinate of all gravity centers, the left-most center of motion gravity 400, this being the gravity center with the lowest x coordinate of all gravity centers, the highest center of motion gravity 402, this being the gravity center with the largest y coordinate of all gravity centers, and the global center of motion gravity, this being calculated based on the median of the x coordinate of the end point of all valid trajectories in the scene over all entities.

Trajectories are defined as trajectories of movement of objects in a video scene which are calculated based on changes in contrast of specific points in the scene. Those points are points which are indicated as possible starting trajectory points. The contrast variability of a point in the scene determines whether that point becomes a starting trajectory point. Starting by a candidate starting trajectory point the gradient in x- and $\gamma$-direction in the image is calculated giving a direction of movement from that starting point. In this way the next trajectory point is determined. A trajectory is formed during a given number of video frames (e.g. 15 frames). When a trajectory has been formed during that number of frames a check is performed to accept or reject the trajectory as a valid trajectory. The validity of a trajectory can be done based on typical characteristics of a logical movement of an object. For instance the length of the trajectory, the curvature of the trajectory, the variation of the trajectory points etc. can be used as quality indicators for the acceptance of a trajectory.

An entity hereinafter is defined as a set of trajectories or a set of moving points in a scene with common characteristics. Clustering algorithms, i.e. the well-known k-means clustering can be used to cluster trajectories or moving points based on for instance color, length of trajectory, slope of trajectory, histogram of a trajectory points etc. It is to be noted here that a trajectory is calculated e.g. 15 frames long, but its length depends on the speed of the corresponding moving object. By clustering trajectories or moving points in the scene body parts of the patient but also other moving parts like bed sheets are identified as such due to their common characteristics.

The center of gravity values are based on the video information. Actually the gravity values are a different and simpler algorithm to cluster moving points and/or trajectories. The median value of the x-coordinates of all the moving points or starting points of trajectories in a region of interest 202, 203 (described below) in the scene is calculated to come with the x-coordinate value of the center of gravity 400, 401. Comparable, the median value of the γ-coordinates of all the moving points or starting points of trajectories in a region of interest 202, 203 in the scene is calculated to come with the γ-coordinate value of the center of gravity 402. In addition a part of the moving points in a region of interest 202, 203 in the scene can be used to calculate the center of gravity 400, 401, 402 of specific subparts of objects in a region of interest 202, 203, i.e. taking only the 30% of the points on the right side of the global gravity point of a window in the scene.

Based on the coordinates of the bed edges 201a, 201b, 201c, 201d, the algorithm defines the two regions of interest 202 and 203a and 203b mentioned above. The first region of interest 202 is of rectangular shape, with a lower base 204 stretching horizontally across a middle section of the bed 200. The horizontal lower base 204 does not exceed in length the width of the bed 200. In vertical direction the first region of interest 202 stretches beyond a top bed edge 201a, with the top bed edge 201a being placed at the middle of the first region of interest 202 in vertical direction.

The second region of interest 203a and 203b is also essentially rectangular in shape, with a lower base 205 stretching horizontally and essentially parallel to the lower base 204 of the first region of interest 202 across the middle section of the bed 200. The horizontal base 205 exceeds the width of the bed 200 by approximately half of the width of the bed 200. In vertical direction the region stretches beyond the top bed edge 201a, with the top bed edge 201a being placed at the middle of the first region of interest 202 in vertical direction. The total size of the second region of interest 203a and 203b might be of approximately the same size as the first region of interest 202. However, any suitable size for the regions of interest 202 and 203 can be chosen.

In the following the method is described by which the video component detects automatically the parameters assigned to the different movement types, based on the regions of interest 202, 203 and the centers of motion gravity 400, 401, 402. The result of each parameter detection step is the value of an indicator α calculated according to the design of each parameter. The α value is translated into an individual visual output indicator UI_RGB 300 for each of the detectable parameters (restless, bed exit, torso up etc.) in the range of green to red, which is an [R, G, B] tuple according to the following formula:

$$UI\_RGB = [\alpha * 255, (1 - \alpha) * 255, 0].$$

The output indicator value is used for display in a user interface of the video component. The user interface is not explicitly shown in Fig. 1, but can be any suitable display unit like a screen or the like. In that sense, if the UI_RGB indicator 300 of a specific parameter is closer to red, the parameter is detected to be more prominently present in the frame covered by the camera, e.g. the patient 2 is detected to be closer to the bed edge 201a, 201b, 201c or 201d or the patient 2 is very restless. Conversely, if the UI_RGB indicator 300 of a specific parameter is closer to green, the parameter is detected to be less prominently present in the frame covered by the camera, e.g. the patient 2 is detected to be further away from the bed edge 201a, 201b, 201c 201d or the patient 2 is lying quite still in bed.

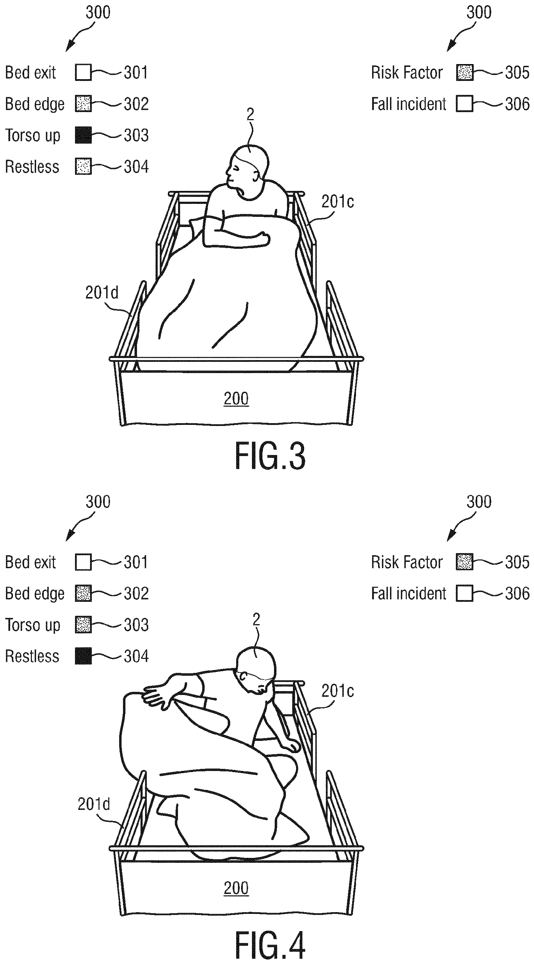

In Figs. 3 to 10, the frame covered by the camera is shown under different conditions of the patient 2. In the display the respective UI_RGB indicators 300 are exemplary arranged in two columns on the left and right sides of the display, with the left side containing an indicator 301 for a "bed exit" event, an indicator 302 for a movement of the patient towards the "bed edge" 201a, 201b, 201c, 201d, an indicator 303 for the upright "torso up" posture and an indicator 304 for "restlessness". On the right hand side an indicator 305 for the video data "risk score" 110 and an indicator 306 for a "fall incident" are arranged. Of course, any other suitable number and arrangement of the UI_RGB indicators 300 is possible. Further, to represent the colors of the UI_RGB indicators 300 clearly in black and white in the figures, an empty square for any of the indicators 300 represents green, while a rising number of dots in the square represents a shift towards red via yellow and orange. A filled square for each of the indicators 300 represents a red color.

Based on the aforementioned regions of interest 202, 203, the video component detects that the patient's torso is up from a lying position when the highest center of motion gravity 402 is located higher than a specific threshold. This specific threshold is below the top boundary of the first region of interest 202 in Fig. 2. This threshold is learned over a set of videos. The situation with the patient's torso being in an upright position is shown in Fig. 3.

The threshold is used to distinguish between a moving point in a scene that appears to be moving due to noise from moving points in the scene that are really representing points of moving objects in the scene. The value of the threshold is calculated by estimating the noise level of the video. That happens based on the average noise level of specific points in the scene when no moving objects are located at those locations in the image. Exemplary, five points at the upper part of a frame are used to calculate the noise of the video, but any set of points scattered over the scene can be used for this calculation. The threshold is calculated in a number of steps.

In case a point which is used to calculate the threshold value is not a point of a moving object in the scene then the variance of the intensity of the point is calculated taking the values of the intensity of the point over the last i.e. 60 frames.

A point which is used to calculate the threshold value of the video is assessed in relation to whether moving objects are located at that point in the scene. That happens by monitoring the intensity change of the point. In case the change of the average value of the intensity of the point, calculated over i.e. the last 60 frames of the video, indicates an abrupt change then the variance of that point over i.e. the last 60 frames of the video is not taken into account in the calculation of the threshold value of the video at that moment of time.

The points that are not rejected based on the considerations described above are used to calculate the variance of the noise level of the video at that moment of time. The variance of the noise level of the video at that moment of time depends on the variance of the points used to calculate the threshold value of the scene. That dependency can be the average of the variances or the maximal value of variances.

The threshold value of the video at that moment of time is then calculated as a multiple of the standard deviation (square root of the aforementioned assessed variance of the video at that moment of time). The multiplication factor is set at 4 (four standard deviations) but it will be based on a training algorithm when field data are available. The training algorithm will be based on training an iterative learning system while using the real videos from the clinical tests.

As can be further recognized in Fig. 3, not only the indicator 303 for the "torso up" posture becomes red when the highest center of motion gravity 402 is higher than the threshold, but also the respective indicator 305 indicating the video data risk score 110 has changed from green to orange, indicating that a fall from the bed 200 might be possible. Likewise, the indicator 302 for approach to the bed edge 201a, 201b, 201c, 201d has changed towards orange since the patient 2 not only sits up but also has shifted slightly to one side of the bed 200. The indicator 304 for restlessness is also slightly away from green, since sitting up will imply a certain amount of movement.

Referring now to Fig. 4, the video component can calculate the variance of the motion of the global center of motion gravity in horizontal direction over a number of consecutive frames, e.g. in a 2 second time interval, to detect restlessness. If this variance is higher than a threshold which can be determined via learning, the video component detects that the patient 2 is restless. The indicator 304 for restlessness in Fig. 4 becomes red when the afore-mentioned variance is higher than the threshold. The further away the variance is from the threshold the further away from red and closer to green the indicator 304 becomes, indicating that the patient 2 is resting quietly. Additionally, the indicators 303 and 302 for "torso up" posture and for the approach of the patient to the bed edge 201 a, 201b, 201c, 201d are changing from green towards orange or red, as does the indicator 305 for the video data risk score 110.

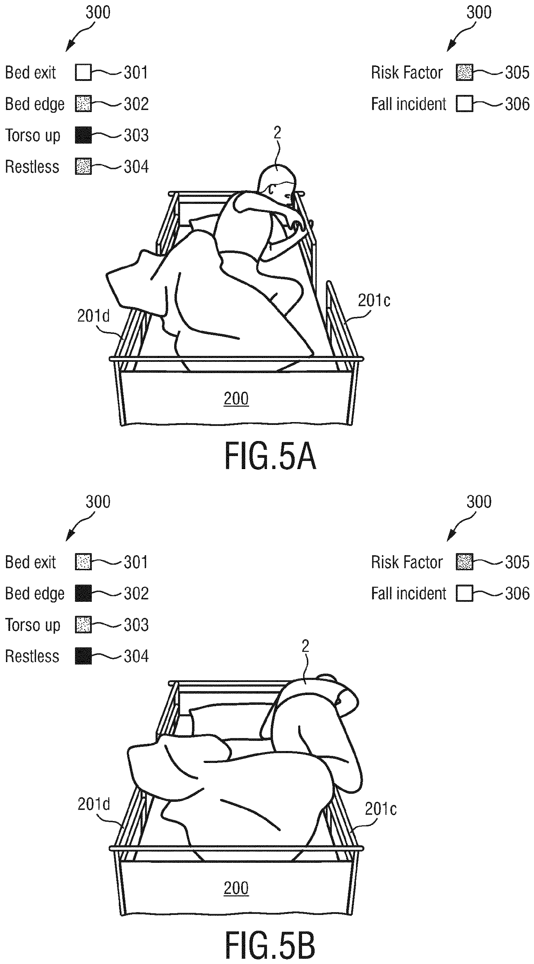

According to Fig. 5A and 5B, the video component further provides a gradual indicator 302 of the patient's position relative to the vertical bed edges 201c, 201d. In that sense monitoring the patient location relative to the bed edge 201c on the right side is done by monitoring the position of the right most center of motion gravity 401 relative to the right vertical edge of the first region of interest 202 in Fig. 2.

Conversely the video component monitors the patient location relative to the bed edge 201d on the left side by monitoring the position of the left most center of motion gravity 400 relative to the left vertical edge of the first region of interest 202. The values of the indicator 302 are closer to green when the respective center of motion gravity 400, 401 is further away from the vertical edge of the first region of interest 202 on the same side, and is closer to red when the center of motion gravity 400, 401 is closer to that same edge.

Accordingly, Fig. 5A shows the indicator 302 on orange as the patient 2 is touching the bed edge 201c while in Fig. 5B the indicator 302 is red as the patient 2 is climbing on or over the bed edge 201c. Due to the mixture of different movements involved, again other indicators 300 are turning their colors. While in Fig. 5A the "bed exit" indicator 301 is still green as the patient 2 is still in bed 200, the respective indicator 301 turns orange in Fig. 5B when the patient 2 is leaving the bed 200. The indicator 305 for the video data risk score 110 is orange, too. The restlessness indicator 304 is likewise away from green as the action of the patient 2 involves a lot of movement.

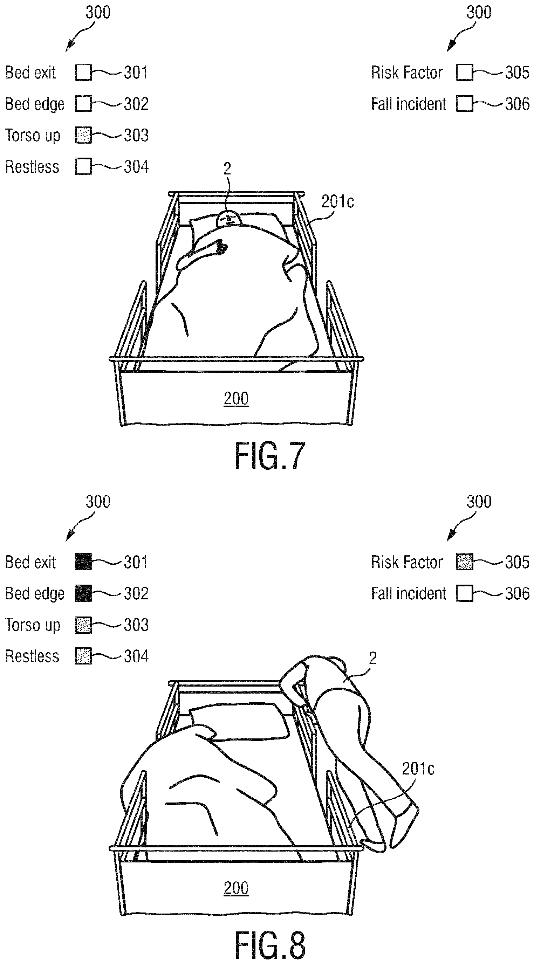

Additionally, the video component can detect that the patient 2 is safe in bed 200 without much movement, e.g. while sleeping or being sedated, when both horizontal centers of motion gravity (right most and left most) 400 and 401 are within the vertical boundaries of the first region of interest 202. This is shown in Fig. 7, where the respective indicators 300 are all green as the patient 2 is not moving.

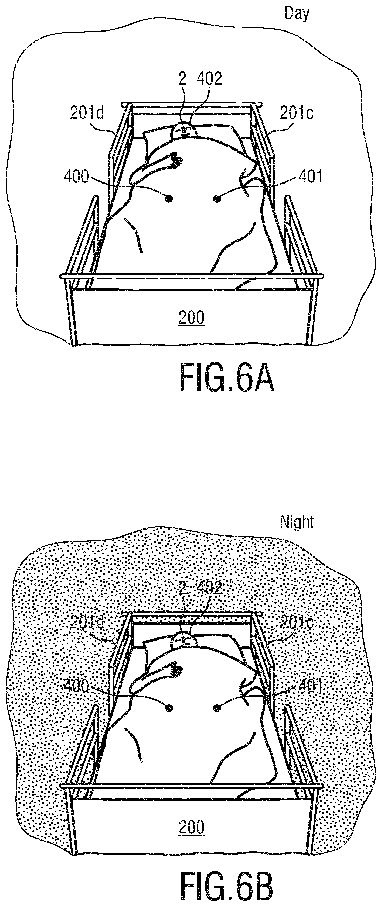

Fig. 6A and 6B illustrate the video component execution in daylight and night time conditions, where the patient 2 is detected to be safely in bed. No indicators 300 are shown in this case. However, the dots 400 and 401 on the blanket covering the patient 2 represent the two horizontal centers of motion gravity, both found within the confines of the bed edges 201c, 201d, that is, within the vertical boundaries of the first region of interest 202.

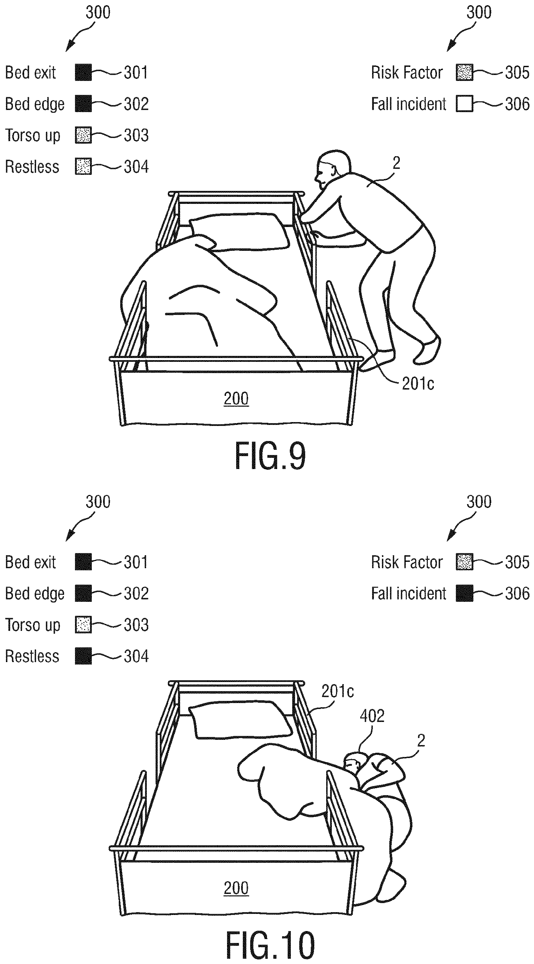

The video component detects that the patient 2 has exited the bed 200 intentionally when the left most center of motion gravity exceeds the right vertical edge of the first region of interest 202 and the right most center of motion gravity 401 exceeds the right vertical edge of the second region of interest 203b. When the aforementioned conditions are met the indicator 301 for "bed exit" becomes red as illustrated in Fig. 8 and 9. As already detailed above, other indicators 300 may change color in this case, since leaving the bed 200 will involve a lot of movement like bringing up the torso, moving towards the bed edge 201 c and thus also triggering the video data risk score 110 to rise.

The video component detects that the patient 2 has fallen out of bed 200 when the left most center of motion gravity 400 exceeds the right vertical edge of the first region of interest 202 and the right most center of motion gravity 401 exceeds the right vertical edge of the second region of interest 203b, when the highest center of motion gravity 402 is located below a certain horizontal threshold placed below the bases 204 and 205 of the regions of interest 202 and 203. The threshold is determined via learning over a number of videos. When the aforementioned conditions are met the indicator 306 for "bed fall" becomes red as illustrated in Figure 10. Other indicators 300 are involved again and change color, only the "torso up" indicator 303 is green since the patient 2 is now lying outside the bed 200, but with torso down.

The calculation of the video data risk score 110 indicating the allover bed fall risk is based on a linear combination or sum of all the detected parameters except fall detection, divided by the number of risk factors detected (No_RF) taken into calculation. In the examples shown in the Figs. 3 to 10, the number of risk factors is three (No_RF = 3):

$$\text{Video data risk score} = (\text{Torso Up} + \text{Restless} + \text{Bed Edge}) \, / \, \text{No\_RF}$$

In the following, the functionality of the vitals signs sensor 13 is described in detail. In the following, analogous to the video component, the vitals signs sensor 13 and the respective vital signs processing unit 9 are termed "vital signs component". The processing unit 9 receives input from the vital signs sensor 13 in the patient room 40 as described with reference to Fig. 1 above.

This part of the device 1 receives input from the vital signs sensor 13 which is arranged in the patient room 40 or directly on the body of the patient like e.g. a pulsemeter, a device for monitoring blood pressure or the like. Alternatively, the vital signs sensor 13 can be a remote sensor, e.g. a PPG sensor, which can monitor the blood oxygen content by way of irradiating certain skin areas. The vital signs component again like the video component combines two functions. On the one hand, the reliability of the vital signs or physiological signals (e.g. heart signal, respiration signal, accelerometer signals etc.) is assessed, on the other hand, the system detects in real time the presence of risk factors based on which it calculates a risk score indicating the likelihood of a bed fall incident. This functionality is executed only if the reliability of the physiological signals as mentioned above is determined to be sufficient.

The modifiable risk factors detected in real-time are the following: the patient experiences tension or stress, the patient experiences anxiety, the patient experiences restlessness, i.e. is tossing and turning, or the patient experiences agitation, which might be expressed via intense or fast, large, erratic movements. Further, the patient's posture in bed space can be detected: patient's torso is up, the patient is leaning over the bed edge or the patient is reaching out of bed.

Detecting the modifiable risk factors above allows calculating a vital signs risk score 111 for a bed fall incident based on the input from the vital signs sensor 13. The Vital signs risk score 111 in the following is denoted with R_VitalSigns. If the vital signs sensor is not reliable the system 11 does not process the signal at all and R_ VitalSigns is set on -1 to indicate that the risk has not been assessed due to signal unreliability.

The assessment of the reliability of the physiological signals received from the vital signs sensor is done by way of a variable to express the vital signs sensor reliability SR_VS. SR_VS is set on 1 when the sensor reliability is assessed to be sufficient and 0 otherwise.

The assessment is based on the signal broadcasting rate as well as on the rate of signal artefacts. In that sense whenever the signal broadcasting rate is below a threshold defined as system requirements (e.g. 1Hz), the signal of the vital signs sensor 13 is determined to be not reliable and SR_VS is set to 0. Similarly, if the rate of signal artefacts is above a threshold, e.g. the proportion of outlier samples within a moving time window is higher than a threshold, that is, for instance, more than 10% of samples are outliers within a 1 minute window, the signal of the vital signs sensor 13 is determined to be not reliable and SR_VS is set to 0.

If however the signal broadcasting rate as well as the signal artefacts rate is within acceptable ranges relative to the thresholds mentioned above, SR_VS is set to 1.

This physiological signals reliability assessment could be done either punctually with a certain frequency or at certain intervals or continuously using a moving signal window. The latter option implies buffering small portions of the signals to be assessed and helps to streamline processor performance.

In the following, the detection of risk factors and calculation of the risk score based on the vital signs data 140 is described.

The vital signs component executes on the dedicated processing unit 9 and receives input from the vital signs sensor 13 based on which it detects in real-time a number of risk factors known to lead to bed-fall incidents as mentioned above. The component uses the detection of the risk factors above to calculate a vital signs risk score R VitalSigns 111 indicating in real time the chance for a bed fall incident. The output of the respective component thus includes the SR_VS signal reliability indicator and the R_ VitalSigns risk score 111.

If the patient 2 experiences some tension or stress, the respective risk factor is detected by analyzing the heart rate (HR), heart rate variability (HRV), respiration rate (RSP Rate) and accelerometer (ACC) signals in the following manner: the system detects within a time window longer than a threshold (e.g. 0.5 mins) an increasing trend of slightly elevated average values relative to the baseline average (e.g. less than 5% increase) for HR, RSP Rate, and slightly lower values for HRV, but the system 11 does not detect significant movement in the ACC signal. The accelerometer preferably is arranged on the patient's 2 chest, but can also be arranged on other suitable locations of the body.

If the patient 2 experiences anxiety, the system detects within a time window longer than a threshold (e.g. 0.5 mins) an increasing trend of significantly elevated average values relative to the baseline average (e.g. larger than 5% increase) for HR, RSP Rate, and lower values for HRV, but the system does not detect significant movement in the ACC signal.

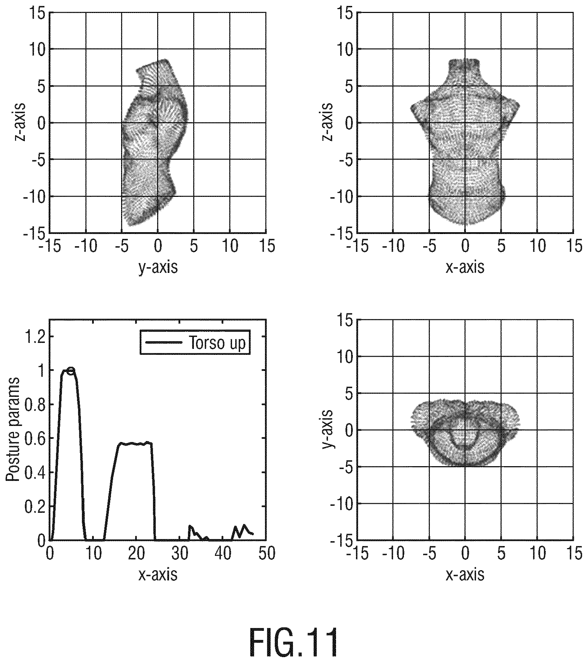

Further, the patient's posture in bed 200 is used to determine a respective risk factor as describe with reference to Figs. 11 to 15.

In case the patient 2 sits up in the bed 200, the "Torso up" signal from Fig. 11 is calculated by applying the following formula (1) to the x, y, z accelerometer signals:

$$\text{Torso up} = \sin(\varphi) = \frac{-tn_y}{\sqrt{tn_x^2 + tn_y^2 + tn_z^2}} \qquad (1)$$

with

$$tn_i = \frac{lcounts_i - cming_i}{cplusg_i - cming_i} \cdot 2 - 1$$

Therein, lcounts$_i$ (i representing the x, y, z, channel number) are low-pass filtered accelerometer counts, cutoff = 1Hz, cming$_i$ = lcounts for -g acceleration, cplusg$_i$ = lcounts for +g acceleration.

The preceding formula is used for normalization of the accelerometer counts "lcounts" to yield normalized values "tn" ranging from "-1" (if the gravity acceleration is pointing into the negative coordinate direction) to "+1" (if the gravity acceleration is pointing into the positive coordinate direction). The "+x" direction points from the patient's chest towards his left side, the "-x" direction towards his right side. The "+y" direction points towards his head, the "-y" direction towards his feet. The "+z" direction points outward from the chest, the "-z" direction points from his chest towards his back. Example: A normalized accelerometer reading of (0; 0; -1) of a patient in rest would indicate a "supine" position, whereas a reading of (0; -1; 0) would indicate a "torso up" position.

The system 11 detects that the patient 2 has his torso up when the Torso Up signal reaches a peak value larger than 0.7 as illustrated in Fig. 11. The round marker in the bottom left part of Fig. 11 indicates the current "torso up" situation illustrated along the three axes in the remaining three parts of Fig. 11.

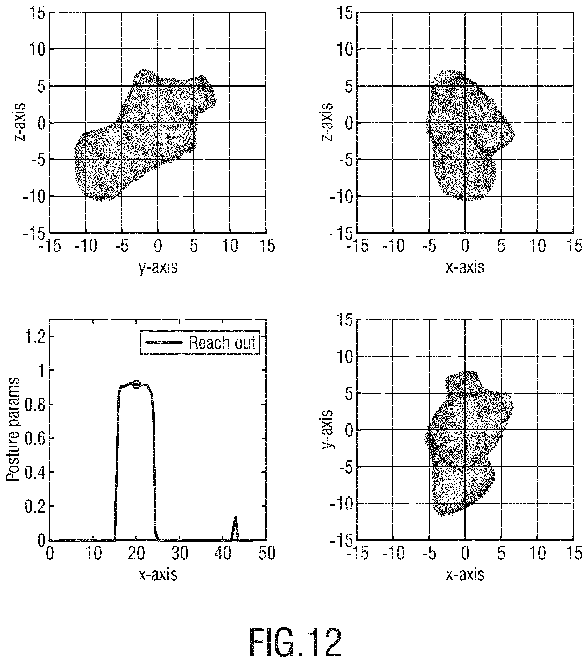

If the patient reaches out of the bed, the "Reach out" signal from Fig. 12 is calculated by applying formula (2) to the x, y, z accelerometer signals:

$$Reach\ out = |\sin(2\psi)| \cdot (tn_z > 0) \cdot \sin(2\varphi) \cdot (tn_y < 0) \qquad (2)$$

with

$$\sin(2\psi) = \frac{2 \cdot |tn_x \cdot tn_z|}{tn_x^2 + tn_z^2}$$

and

$$\sin(2\varphi) = \frac{-2 \cdot tn_y \cdot \sqrt{tn_x^2 + tn_z^2}}{tn_x^2 + tn_y^2 + tn_z^2}$$

The first factor in "Reach out" (=|sin(2$\psi$)|*(t$_z$>0)) is thus at maximum (=1) if $\psi$=45deg and t$_z$>0. The second factor in "Reach out" (=sin(2($\phi$)*(t$_y$<0)) is at maximum (=1) if $\phi$=45deg and t$_y$<0.

The system 11 detects that the patient 2 is reaching out when the Reach Out signal reaches a peak value larger than 0.8 as illustrated in Fig. 12. The round marker in the bottom left part of Fig. 12 indicates the current "reach out" situation illustrated along the three axes in the remaining three parts of Fig. 12.

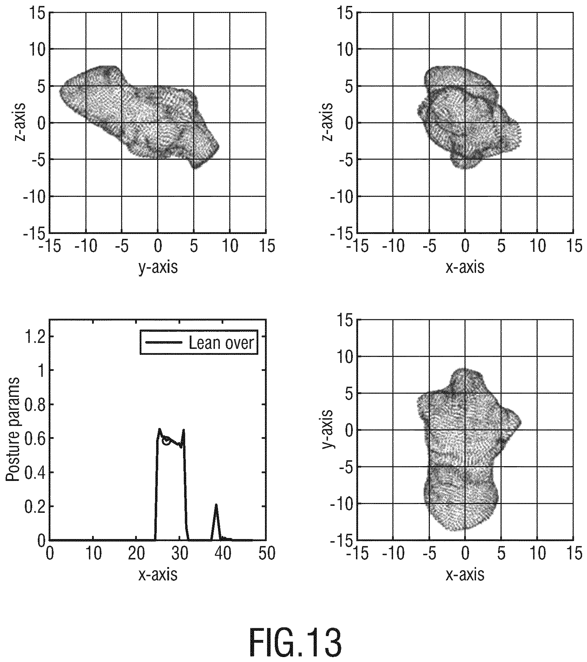

If the patient 2 is leaning over the bed edge 201, the "Lean over" signal from Fig. 13 is calculated by applying formula (3) to the x, y, z accelerometer signals:

$$Lean\ over = |\sin(2\psi)| \cdot (tn_z > 0) \cdot -\sin(2\varphi) \cdot (tn_y > 0) \qquad (3)$$

with

$$\sin(2\psi) = \frac{2 \cdot |tn_x \cdot tn_z|}{tn_x^2 + tn_z^2}$$

and

$$\sin(2\varphi) = \frac{-2 \cdot tn_y \cdot \sqrt{tn_x^2 + tn_z^2}}{tn_x^2 + tn_y^2 + tn_z^2}$$

The first factor in "Lean over" (=|sin(2$\psi$)|*(t$_z$>0)) is thus at maximum (=1) if $\psi$=45deg and t$_z$>0. The second factor in "Lean over" (=-sin(2$\phi$)*(t$_y$>0)) is at maximum (=1) if $\phi$=-45deg and t$_y$>0.

The system 11 detects that the patient 2 is leaning over when the Lean Over signal reaches a peak value larger than 0.5 as illustrated in Fig. 13. The round marker in the bottom left part of Fig. 13 indicates the current "lean over" situation illustrated along the three axes in the remaining three parts of Fig. 13.

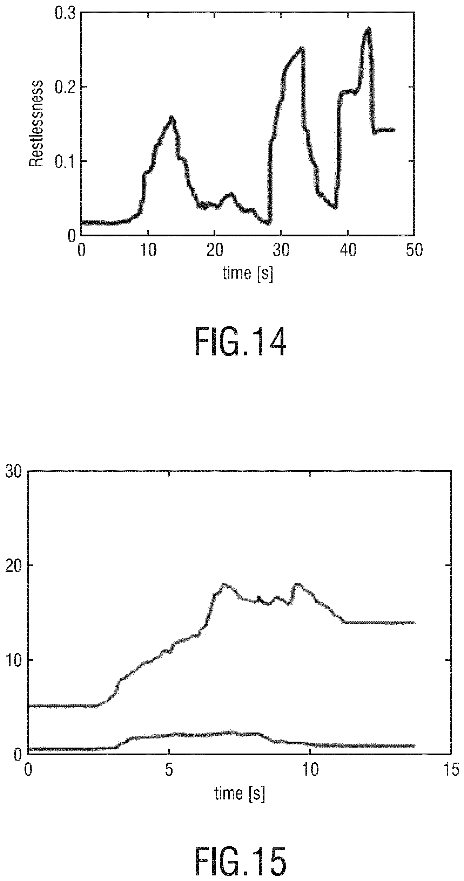

Restlessness is defined as a motion where the patient torso is orientated horizontally, and movement amplitude is limited and predominant on the x and z axes referring to tossing and turning movements of the body. If the patient 2 experiences restlessness, the "restlessness" signal illustrated in Fig. 14 is obtained by applying formula (4) to the x, y, z accelerometer signals.

$$Restlessness = movav(50 \cdot rm, 5s) \qquad (4)$$

with $rm = \sqrt{rn_x^2 + rn_y^2 + rn_z^2} \cdot (1 - TorsoUp),$ where Torso Up is defined in formula (1) above and

$rn_i = \frac{hcounts_i}{cplusg_i - cming_i} \cdot 2$ where movav(A,T) is a moving average of A over time T, hcounts$_i$ represents a high-pass filtered accelerometer counts (i is x, y, or z accelerometer channels), with cutoff =2Hz.

The quantity "rni" are normalized, high-pass filtered accelerometer signals for each coordinate direction i. Then the magnitude of the vector given by the individual components rni is calculated, i.e. a quantity indicating rapidly varying accelerations of the patient 2. But the patient 2 could also perform "normal" activities like walking, running or jumping. All these "normal" activities would be characterized by a "TorsoUp" signal close to 1, whereas "restlessness" of a patient lying in bed would be characterized by a "TorsoUp" signal close to 0. Therefore, the definition of "rm" includes a factor "(1-TorsoUp)" to suppress the mentioned "normal" activities in the calculation of "restlessness". During phases of restlessness the signal "rm" is rapidly oscillating between 0 and its maximum value, therefore finally a "moving average" operation "movav" is performed, i.e. averaging the signal "rm" (multiplied by an arbitrary factor 50) over the last 5s to obtain the "Restlessness" signal.

In Fig. 14, restlessness is expressed via a signal obtained by means of applying formula (1) to the x, y, z accelerometer signals.

Patient agitation is defined as a motion where the patient torso is orientated vertically, and movement amplitude is high, especially significantly higher than in the case of restlessness as in Figure 14, and predominant on the y and x axes. Agitation is expressed via intense / fast, large, erratic movements.

This is modeled by the following formula (5)

$$Agitation = movav(50 \cdot rm, 5s) \tag{5}$$

with $rm = \sqrt{rn_x^2 + rn_y^2 + rn_z^2} \cdot TorsoUp,$ where Torso Up is defined in formula (1) above and

$$rn_i = \frac{hcounts_i}{cplusg_i - cming_i} \cdot 2$$

where movav(A,T) is a moving average of A over time T, hcounts$_i$ represents a high-pass filtered accelerometer counts (i is x, y, or z accelerometer channels), with cutoff =2Hz.

In Fig. 15, a combined illustration of restlessness and agitation signals shows the amplitude of movement at agitation being significantly higher than in the case of restlessness (upper graph in Fig. 15).

Calculation of the R _VitalSigns risk score 111 is based on assessing the risk factors above, where the R_VitalSigns is increased by a parameter xi (i= 1..8) - associated with each risk factor. This would imply the following tests and associated computations:

If Stress detected = yes then
Risk Score = Risk Score $\otimes$ x1

If Anxiety detected = yes then
Risk Score = Risk Score $\otimes$ x2

If Patient TorsoUp detected = yes then
Risk Score = Risk Score $\otimes$ x3

If Patient ReachOut detected = yes then
Risk Score = Risk Score $\otimes$ x4

If Patient Lean Over detected = yes then
Risk Score = Risk Score $\otimes$ x5

If Patient Restlessness detected = yes then
Risk Score = Risk Score $\otimes$ x6

If Patient Agitation detected = yes then
Risk Score = Risk Score $\otimes$ x7

wherein in a simple example "$\otimes$" could stand for a simple sum.

There are, however, other possibilities to connect the risk scores. The actual formula will be learned from the data, i.e. from the different occurrence of the single parameters.

At system initialization parameters x1 to x7 can be initialized based on values indicated by literature studies, while over time, the system 11 adjusts the parameters values by learning the influence of each risk factor above on a bed fall occurrence, for the specific population at hand. This is done by executing correlation analyses and applying regression techniques after each bed fall incident to understand the impact of each risk factor on the likelihood of a bed fall event and to update the values of the parameters above. As time passes the system 11 will thus become increasingly accurate in determining the risk score. If no bed fall incident occurs no parameter adjustment is needed.

In the following, the intervention unit 15 will be described in more detail. The intervention unit 15 preferably is located in the workstation room 50 away from the patient rooms 40 to allow access for the medical staff and the maintenance staff alike and to allow monitoring of many individuals simultaneously. The intervention unit 15 receives as input the patient profile from the classification unit 10 and the total risk score 114 as described above calculation unit 6.

Based on those values (e.g. total risk score 114 is above a threshold or increasing significantly within a short time window) the intervention unit 15 performs one or more of the following actions:
Either it contacts the patient via the feedback unit 14 in the patient room 40 and assesses the situation, attempts to calm the patient 2 if necessary, persuades the patient 2 to remain in bed 200, while medical staff is on the way to attend to the patient's needs.

Alternatively or simultaneously, the intervention unit identifies the nearest available medical staff and sends the above information to that staff member to ensure speed of action. In order to identify the staff nearest to the patient room 40 the intervention unit 15 may use locating technologies (e.g. GPS). In order to identify the staff available at the time, the intervention unit 15 consults a staff schedule 17 which might be stored in a database indicating the staff availability at that time.

The intervention unit 15 in any case ends the medical staff an alarm code indicating risk for bed fall incident with intervention needed.

Intervention information indicating details regarding the patient profile and the recommended intervention for this particular patient given profile may also be sent by the intervention unit 15.

Besides, the intervention unit 15 informs medical staff regarding the timeframe within an intervention needs to be provided in order to be effective and prevent the incident.

Further, the intervention unit 15 helps prioritize the current patient needs above other prospective activities.

The intervention unit 15 is part of the larger infrastructure responsible for patient monitoring, bed fall risk assessment, patient classification based on profile and adaptive intervention as described in Fig. 1.

The intervention unit 15 makes use of an artificially intelligent implementation that employs e.g. speech recognition for direct communication with the patient 2 when needed. The system distinguishes 3 situations relevant in this context:

1. The total risk score 114 is low or moderate (below a certain threshold) but slow to moderately increasing within a limited time-window - e.g. risk score values rise under an average slope of a maximum 10 degree angle (average slope <= 0.17) during the last 10 minutes.

2. The total risk score 114 is low or moderate (below a certain threshold) but significantly increasing within a limited time-window (e.g. 5-10 minutes) - e.g. risk score values rise under an average slope of an angle larger than 10 degrees (average slope > 0.17).

3. The total risk score 114 is determined to be high (above a certain threshold).

The intervention unit 15 reacts in each situation described above based on information derived from the patient profile. In that sense, the system classifies the patient 2 in terms of Cognitive Ability Level (CgAL), Communication Ability Level (CmAL) and Compliance Level (CL). If based on the patient profile, patient does not have a cognitive impairment and no speech impairment CgAL is set to High. In any other situation it is set to Low. In addition, based on the patient profile (specifically the psychological compliance profile) the system sets CL to High or Low.

In the following tables, the situations by which the intervention unit 15 reacts to the value trends of the total risk score 114 are described.
1. If the total risk score 114 is low or moderate (below a certain threshold) but slow to moderately increasing within a limited time-window (e.g. 10 minutes), the intervention unit 15 makes a projection based on current risk score growth how long until the total risk score 114 will become high to indicate to the nurse the time frame available for an effective prevention. This will help with prioritizing the patients that need attendance.

Table 1

| No | CgAL | CmAL | CL | eSitter intervention |
|----|------|------|-----|----------------------|
| 1 | High | High | High | Intervention unit 15 communicates with patient 2 e.g. via audio: enquires patient if he / she has needs that must be attended to, records patient response (AV stream), gently suggests patient 2 to wait safely in bed, and that a nurse on duty is informed right away. Intervention unit 15 contacts the nurse on duty and sends to their mobile phone a message specifying that bed fall risk grows, plays the record of the patient indicating their needs, indicates to nurse the time frame she should attend to patient need in order to prevent an incident. |

(continued)

| No | CgAL | CmAL | CL | eSitter intervention |
|----|------|------|-----|----------------------|
| | | | | Intervention unit 15 keeps in touch reassuring patient 2 by updating on how long it takes until nurse attends to needs. |
| 2 | High | High | Low | Intervention unit 15 communicates with patient 2 e.g. via audio: enquires patient if he / she has needs that must be attended to, records patient response (AV stream), advises patient 2 to wait safely in bed, and that a nurse on duty is informed right away.<br>Intervention unit 15 contacts the nurse on duty and sends to their mobile phone a message specifying: bed fall risk grows, patient 2 is non-compliant, plays the record of the patient 2 indicating their needs, indicates to nurse the time frame she should attend to patient need in order to prevent an incident. Intervention unit 15 keeps in touch with patient 2 by updating on how long it takes until nurse attends to needs. |
| 3 | High | Low | High | Intervention unit 15 communicates with patient 2 e.g. via audio and gently suggests patient 2 to wait safely in bed, and that a nurse on duty is informed right away.<br>Intervention unit 15 contacts the nurse on duty and sends to their mobile phone a message specifying: bed fall risk grows, patient 2 is not able to verbally communicate, indicates to nurse the time frame she should attend to patient need in order to prevent an incident.<br>Intervention unit 15 keeps in touch reassuring patient 2 by updating on how long it takes until nurse attends to needs. |
| 4 | High | Low | Low | Intervention unit 15 communicates with patient 2 e.g. via audio and gently suggests patient 2 to wait safely in bed, and that a nurse on duty is informed right away.<br>Intervention unit 15 contacts the nurse on duty and sends to their mobile phone a message specifying: bed fall risk grows, patient 2 is not able to verbally communicate, patient 2 is non-compliant, indicates to nurse the time frame she should attend to patient need in order to prevent an incident.<br>Intervention unit 15 keeps in touch reassuring patient 2 by |
| | | | | updating on how long it takes until nurse attends to needs. |
| 5 | Low | High | High | Intervention unit 15 communicates with patient 2 e.g. via audio, addressed patient 2 gently indicating medical staff will attend very soon (AV stream). Additional calming stimuli could be provided (e.g. gentle nature sounds or music), while intervention unit 15 maintains touch with patient and keeps reassuring.<br>Intervention unit 15 contacts the nurse on duty and sends to their mobile phone a message specifying: bed fall risk grows, patient 2 is cognitively impaired, patient 2 can be communicated with, indicates to nurse the time frame she should attend to patient need in order to prevent an incident, indicates to nurse she needs to give this patient 2 even higher priority given higher unpredictability induced by cognitive impairment, recommends staff on ways of approaching the patient given cognitive impairment, that likely patient 2 might start feeling disoriented, confused and/or anxious. |
| 6 | Low | High | Low | Similar to No. 5. except that medical staff is informed patient 2 is not compliant. |
| 7. | Low | Low | High | Similar to No. 5. except that medical staff is informed patient 2 cannot communicate. |
| 8. | Low | Low | Low | Similar to No. 5. except that medical staff is informed patient 2 cannot communicate and is not compliant. |

2. If the total risk score 114 is low or moderate (below a certain threshold) but significantly increasing within a limited time-window (e.g. 5-10 minutes), the intervention unit 15 again makes a projection based on current risk score growth how long until the total risk score 114 will become high to indicate to the nurse on duty or nearest available medical staff the time frame available for an effective preventive intervention. This will help with prioritizing the patients 2 that need attendance.

The intervention matrix is similar to the one specified in Table 1 with the difference that in this case the patient automatically becomes highest priority for nurses on duty, and if all nurses on duty engaged with other patients 2 and unable to attend the nearest available medical staff is contacted.

3. If the total risk score 114 is determined to be high (above a certain threshold), differences with regard to the previously described situations occur. Action is immediately required. The intervention unit 15 simply identifies the nearest available medical staff and informs nurses on duty as well that patient needs to be attended.

Table 2

| No | CgAL | CmAL | CL | eSitter intervention |
|---|---|---|---|---|
| 1 | High | High | High | Intervention unit 15 communicates with patient 2 e.g. via audio: enquires reason for impending bed exit and records patient response (AV stream), gently suggests patient 2 to wait safely in bed, and that medical staff will attend his/her need soon. Intervention unit 15 locates nearest available medical staff and sends to their mobile phone a message with high importance specifying: alarm code for bed fall, plays the record of the patient 2 indicating their needs; depending on the need expressed by patient, intervention unit 15 recommends a course of action for the staff to consider. Intervention unit 15 keeps in touch reassuring patient 2 by updating on how long it takes until medical staff arrives. Alternatively the video UI interface could display the hospital map and as a progressing dot the position of the approaching staff to the patient room 40. |
| 2 | High | High | Low | Intervention unit 15 communicates with patient 2 e.g. via audio, emphasizes to patient 2 the necessity to remain in bed (AV stream) until attended due to risk fall. Intervention unit 15 locates nearest available medical staff and sends to their mobile phone a message with high importance specifying: alarm code for bed fall, indicates that patient 2 can be communicated with but has low compliance and immediate attendance is required, recommends staff on ways of approaching the patient regarding persuasion techniques. Intervention unit 15 keeps in touch with patient 2, indicating that the medical staff "will be with you any moment, please do not leave your bed". |
| 3 | High | Low | High | Intervention unit 15 communicates with patient 2 e.g. via audio: gently suggests patient 2 to wait safely in bed, and that medical staff will attend his/her need soon. Intervention unit 15 locates nearest available medical staff and sends to their mobile phone a message with high importance specifying: alarm code for bed fall. Intervention unit 15 keeps in touch reassuring patient 2 by updating on how long it takes until medical staff arrives. Alternatively the video UI interface could display the hospital map and as a progressing dot the position of the approaching staff to the patient room 40. |
| 4 | High | Low | Low | Similar to No. 2. |
| 5 | Low | High | High | Intervention unit 15 communicates with patient 2 e.g. via audio, addressed patient 2 gently indicating medical staff will attend very soon (AV stream). Additional calming stimuli could be provided (e.g. gentle nature sounds or music), while intervention unit 15 maintains touch with patient 2 and keeps reassuring. Intervention unit 15 locates nearest available medical staff and sends to their mobile phone a message with high importance specifying: alarm code for bed fall, indicates that patient can be communicated with but has low cognitive ability and immediate attendance is required, recommends staff on ways of approaching the patient given cognitive impairment, that likely patient 2 feels disoriented, confused and/or anxious. |
| 6 | Low | High | Low | Similar to No. 5. |

(continued)

| No | CgAL | CmAL | CL | eSitter intervention |
|---|---|---|---|---|
| 7. | Low | Low | High | Similar to No. 5. except that medical staff is informed patient 2 cannot communicate. |
| 8. | Low | Low | Low | Similar to No. 5. except that medical staff is informed patient 2 cannot communicate and is not compliant. |

As mentioned before, the feedback unit 14 (referred to by "communicates by audio" in tables 1 and 2) can be a TV set usually available in hospital rooms nowadays. However, if the environment e.g. is a nursing home for cognitively impaired persons the feedback unit can be a display with speakers, microphone and further means of communication arranged in a suitable location near the patient 2.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device (60) for adaptive intervention with an individual (2) in connection with a bed fall risk of the individual (2), the device (60) comprising:

   - a first port (70) for obtaining personal data (120) of the individual (2) and a total risk score (114) of the individual (2),
   - an intervention unit (15) for determining a projection regarding an imminent bed fall of the individual (2) and a timeframe for an intervention to prevent the bed fall of the individual (2) based on the obtained data (120, 114), and
   - a second port (71) for outputting an intervention signal based to the projection and the obtained data (120, 114).

2. Device (60) according to claim 1, wherein the intervention unit (15) is configured to output the intervention signal directly to the individual (2) by way of an interface (14) arranged in the vicinity of the individual (2).

3. Device (60) according to claim 1, further comprising a third port (71) for obtaining data related to a staff schedule (17).

4. Device (60) according to claim 3, wherein the intervention unit (15) is configured to output the intervention signal to a staff member and to trigger a staff intervention at the individual (2) based on the staff schedule (17), the obtained data (120, 114) and the projection.

5. Device (60) according to claim 1, wherein the personal data (120) of the individual (2) comprises one or more of age, gender, neurologic condition, symptoms, disabilities, medication, fall history, psychological profile.

6. Device (60) according to claim 1, wherein the total risk score (114) is determined by obtaining the personal data (120) of the individual (2) and by obtaining sensor data (130, 140) from at least one sensor (12, 13) arranged in the vicinity of the individual (2) and/or in contact with the individual (2).

7. Device (60) according to claim 1, wherein the intervention unit (15) is configured to distinguish distinct ability levels of the individual (2) based on the total risk score (114) and its change and/or development during a predetermined time-frame, wherein the ability levels of the individual (2) comprise a cognitive ability level, a communication ability level, a compliance level.

**8.** Device (60) according to claim 7, wherein the cognitive ability level is related to the total risk score (114) being low or moderate but slowly to moderately increasing within a limited time-window.

**9.** Device (60) according to claim 7, wherein the communication ability level is related to the total risk score (114) being low or moderate but significantly increasing within a limited time-window.

**10.** Device (60) according to claim 7, wherein the compliance level is related to the total risk score (114) being determined to be high.

**11.** System (61) for adaptive intervention with an individual (2) in connection with a bed fall risk of the individual (2), the system comprising:

- a classification unit (10) for providing personal data (120) of the individual (2),
- a calculation unit (6) for calculating a total risk score (114) of the individual (2), and
- a device (60) according to one of the preceding claims for adaptive intervention with the individual (2) based on the output of the classification unit (10) and the calculation unit (6).

**12.** Computer-implemented method for determination of a bed fall risk of an individual, the method comprising the steps of:

- obtaining personal data of the individual, and
- obtaining a total risk score of the individual,
- determining a projection regarding an imminent bed fall of the individual and a timeframe for an intervention to prevent the bed fall of the individual based on the obtained data, and
- intervening at the individual based on the obtained data and the projection.

**13.** Method according to claim 12, wherein the step of intervening at the individual comprises addressing the individual directly via an interface and/or triggering an intervention by staff based on a staff schedule, the obtained data and the projection.

**14.** Method according to claim 12, wherein the step of triggering an intervention by staff comprises one or more of the steps of:

- identifying the nearest available staff member,
- sending an alarm code to the staff member,
- informing the staff member about the available time-frame to prevent a bed fall incident,
- informing the staff about details of the patient profile of the individual,
- informing staff about the recommended intervention action,
- prioritizes the intervention with respect to other tasks of the staff member.

**15.** Computer program comprising program code means for causing a computer to carry out the steps of the method as claimed in claim 12 when said computer program is carried out on a computer.

**Patentansprüche**

**1.** Vorrichtung (60) zur anpassungsfähigen Intervention bei einer Person (2) in Verbindung mit einem Risiko der Person (2), aus dem Bett zu fallen, wobei die Vorrichtung (60) Folgendes umfasst:

- einen erste Anschluss (70) zum Erhalt persönlicher Daten (120) der Person (2) und eine Gesamtrisikoeinstufung (114) der Person (2),
- eine Interventionseinheit (15) zum Bestimmen einer Prognose bezüglich eines bevorstehenden Fallens der Person (2) aus dem Bett und eines Zeitrahmens für eine Intervention zur Verhinderung des Fallens der Person (2) auf Grundlage der erhaltenen Daten (120, 114), und
- einen zweiten Anschluss (71) zur Ausgabe eines Interventionssignals auf Grundlage der Prognose und der erhaltenen Daten (120, 114).

2. Vorrichtung (60) nach Anspruch 1, wobei die Interventionseinheit (15) so konfiguriert ist, dass sie das Interventionssignal direkt an die Person (2) ausgibt via einer in der Nähe der Person (2) angeordneten Schnittstelle (14).

3. Vorrichtung (60) nach Anspruch 1, die ferner einen dritten Anschluss (71) zum Erhalt von Daten im Zusammenhang mit einem Personaleinsatzplan (17) umfasst.

4. Vorrichtung (60) nach Anspruch 3, wobei die Interventionseinheit (15) so konfiguriert ist, dass sie das Interventionssignal an ein Personalmitglied ausgibt und eine Personalintervention bei der Person (2) auf der Grundlage des Personalplans (17), der erhaltenen Daten (120, 114) und der Prognose auslöst.

5. Vorrichtung (60) nach Anspruch 1, wobei die persönlichen Daten (120) der Person (2) eines oder mehrere der Folgenden umfassen: Alter, Geschlecht, neurologisches Befinden, Symptome, Behinderungen, Medikamenten, vorheriges Fallen, psychologisches Profil.

6. Vorrichtung (60) nach Anspruch 1, wobei die Gesamtrisikoeinstufung (114) bestimmt wird, indem die persönlichen Daten (120) der Person (2) und die Sensordaten (130, 140) von mindestens einem Sensor (12, 13) , der in der Nähe der Person (2) und/oder in Kontakt mit der Person (2) angeordnet ist, erhalten werden.

7. Vorrichtung (60) nach Anspruch 1, wobei die Interventionseinheit (15) so konfiguriert ist, dass sie verschiedene Fähigkeitsniveaus des Individuums (2) auf Grundlage der Gesamtrisikobewertung (114) und seiner Veränderung und/oder Entwicklung während eines vorbestimmten Zeitrahmens unterscheidet, wobei die Fähigkeitsniveaus des Individuums (2) ein kognitives Fähigkeitsniveau, ein Kommunikationsfähigkeitsniveau und ein Befolgungsniveau umfassen.

8. Vorrichtung (60) nach Anspruch 7, wobei das kognitive Fähigkeutsniveau mit der Gesamtrisikobewertung (114) in Beziehung steht, die niedrig oder mäßig ist, aber innerhalb eines begrenzten Zeitfensters langsam bis mäßig ansteigt.

9. Vorrichtung (60) nach Anspruch 7, wobei das Kommunikationsfähigkeitsniveau mit der Gesamtrisikoeinstufung (114) in Beziehung steht, die niedrig oder mäßig ist, aber innerhalb eines begrenzten Zeitfensters signifikant ansteigt.

10. Vorrichtung (60) nach Anspruch 7, wobei das Befolgungsniveau mit der Gesamtrisikobewertung (114), die als hoch bestimmt wird, in Beziehung steht.

11. System (61) zur anpassungsfähigen Intervention bei einer Person (2) in Verbindung mit einem Risiko der Person (2), aus dem Bett zu fallen, wobei das System Folgendes umfasst:

    - eine Klassifizierungseinheit (10) zur Bereitstellung persönlicher Daten (120) der Person (2),
    - eine Berechnungseinheit (6) zur Berechnung einer Gesamtrisikobewertung (114) der Person (2), und
    - eine Vorrichtung (60) gemäß einem der vorhergehenden Ansprüche für adaptive Intervention bei einer Person (2) auf Grundlage der Ausgabe der Klassifikationseinheit (10) und der Berechnungseinheit (6).

12. Computer-implementiertes Verfahren zur Bestimmung eines Risikos einer Person aus dem Bett zu fallen, wobei das Verfahren die folgenden Schritte umfasst:

    - Erhalt persönlicher Daten der Person, und
    - Erhalt einer Gesamtrisikoeinstufung der Person,
    - Bestimmung einer Projektion bezüglich eines bevorstehenden Fallens der Person aus dem Bett und eines Zeitrahmens für eine Intervention zur Verhinderung des Fallens der Person auf Grundlage der erhaltenen Daten, und
    - Intervention bei der Person auf Grundlage der erhaltenen Daten und der Prognose.

13. Verfahren nach Anspruch 12, wobei der Schritt der Intervention bei der Person die direkte Ansprache der Person über eine Schnittstelle und/oder das Auslösen einer Intervention durch Personal auf Grundlage eines Personalplans, der erhaltenen Daten und der Prognose umfasst.

14. Verfahren nach Anspruch 12, wobei der Schritt des Auslösens einer Intervention durch das Personal einen oder mehrere der folgenden Schritte umfasst:

- Identifizierung des nächstgelegenen verfügbaren Mitarbeiters,
- Senden eines Alarmcodes an den Mitarbeiter,
- Informieren des Mitarbeiters über den verfügbaren Zeitrahmen zur Verhinderung des Fallen aus dem Bett,
- Informieren des Personals über Einzelheiten des Patientenprofils der Person,
- Informieren des Personals über die empfohlene Interventionsmaßnahme,
- Priorisieren der Intervention im Hinblick auf andere Aufgaben des Mitarbeiters.

15. Computerprogramm mit Programmcode, um einen Computer zu veranlassen, die Schritte des Verfahrens nach Anspruch 12 durchzuführen, wenn das Computerprogramm auf einem Computer ausgeführt wird.

**Revendications**

1. Dispositif (60) d'intervention adaptative auprès d'un individu (2) en relation avec un risque de chute du lit de l'individu (2), ledit dispositif (60) comprenant :

   - un premier port (70) pour obtenir des données personnelles (120) de l'individu (2) et un score de risque total (114) de l'individu (2),
   - une unité d'intervention (15) pour déterminer une projection concernant une chute du lit imminente de l'individu (2) et un intervalle de temps pour une intervention pour empêcher la chute du lit de l'individu (2) en fonction des données (120, 114) obtenues, et
   - un deuxième port (71) pour sortir un signal d'intervention basé sur la projection et les données (120, 114) obtenues.

2. Dispositif (60) selon la revendication 1, dans lequel l'unité d'intervention (15) est conçue pour sortir le signal d'intervention directement à l'individu (2) au moyen d'une interface (14) disposée à proximité de l'individu (2).

3. Dispositif (60) selon la revendication 1, comprenant en outre un troisième port (71) pour obtenir des données liées à des horaires du personnel (17).

4. Dispositif (60) selon la revendication 3, dans lequel l'unité d'intervention (15) est conçue pour sortir le signal d'intervention à un membre du personnel et pour déclencher une intervention du personnel auprès de l'individu (2) en fonction des horaires du personnel (17), des données (120, 114) obtenues et de la projection.

5. Dispositif (60) selon la revendication 1, dans lequel les données personnelles (120) de l'individu (2) comprennent l'âge et/ou le sexe et/ou l'état neurologique et/ou les symptômes et/ou les incapacités et/ou les médicaments et/ou les antécédents de chutes et/ou le profil psychologique.

6. Dispositif (60) selon la revendication 1, dans lequel le score de risque total (114) est déterminé par obtention des données personnelles (120) de l'individu (2) et par obtention des données de capteur (130, 140) provenant d'au moins un capteur (12, 13) disposé à proximité de l'individu (2) et/ou en contact avec l'individu (2).

7. Dispositif (60) selon la revendication 1, dans lequel l'unité d'intervention (15) est conçue pour distinguer des niveaux de capacité distincts de l'individu (2) en fonction du score de risque total (114) et de son changement et/ou développement pendant un intervalle de temps prédéterminé, dans lequel les niveaux de capacité de l'individu (2) comprennent un niveau de capacité cognitive, un niveau de capacité de communication, un niveau de conformité.

8. Dispositif (60) selon la revendication 7, dans lequel le niveau de capacité cognitive est lié au score de risque total (114) étant faible ou modéré, mais augmentant lentement à modérément dans un intervalle de temps limité.

9. Dispositif (60) selon la revendication 7, dans lequel le niveau de capacité de communication est lié au score de risque total (114) étant faible ou modéré, mais augmentant de manière significative dans un intervalle de temps limité.

10. Dispositif (60) selon la revendication 7, dans lequel le niveau de conformité est lié au score de risque total (114) déterminé comme étant élevé.

11. Système (61) d'intervention adaptative auprès d'un individu (2) en relation avec un risque de chute du lit de l'individu (2), ledit système comprenant :

- une unité de classification (10) pour fournir des données personnelles (120) de l'individu (2),
- une unité de calcul (6) pour calculer un score de risque total (114) de l'individu (2), et
- un dispositif (60) selon l'une quelconque des revendications précédentes pour une intervention adaptative auprès de l'individu (2) en fonction de la sortie de l'unité de classification (10) et de l'unité de calcul (6).

12. Procédé mis en œuvre par ordinateur pour déterminer un risque de chute du lit d'un individu, ledit procédé comprenant les étapes suivantes :

- l'obtention des données personnelles de l'individu, et
- l'obtention d'un score de risque total de l'individu,
- la détermination d'une projection concernant une chute du lit imminente de l'individu et d'un intervalle de temps pour une intervention pour empêcher la chute du lit de l'individu en fonction des données obtenues, et
- l'intervention auprès de l'individu en fonction des données obtenues et de la projection.

13. Procédé selon la revendication 12, dans lequel l'étape d'intervention auprès de l'individu comprend l'adressage direct de l'individu par l'intermédiaire d'une interface et/ou d'un déclenchement d'une intervention du personnel en fonction des horaires du personnel, des données obtenues et de la projection.

14. Procédé selon la revendication 12, dans lequel l'étape de déclenchement d'une intervention par le personnel comprend les étapes suivantes :

- l'identification du membre du personnel disponible le plus proche,
- l'envoi d'un code d'alarme au membre du personnel,
- l'information du membre du personnel sur l'intervalle de temps disponible pour éviter un accident de chute du lit,
- l'information du personnel sur les détails du profil de patient de l'individu,
- l'information du personnel sur l'action d'intervention recommandée,
- la prioritarisation de l'intervention par rapport aux autres tâches du membre du personnel.

15. Programme informatique comprenant un moyen de code de programme pour amener un ordinateur à mettre en œuvre les étapes du procédé selon la revendication 12 lorsque ledit programme informatique est exécuté sur un ordinateur.

Bed Fall Alarm Code
Intervention Code

Patient Profile

Total Risk Score

Intrinsic Risk Score

Variable Risk Score

BE, BF, AP
RS_V

R_Video
RS_V

R_Vital signs
RS_VS

FIG.1

FIG.2

300

Bed exit ☐ 301
Bed edge ▨ 302
Torso up ■ 303
Restless ▨ 304

300

Risk Factor ▨ 305
Fall incident ☐ 306

2

201c

201d

200

FIG.3

300

Bed exit ☐ 301
Bed edge ▨ 302
Torso up ▨ 303
Restless ■ 304

300

Risk Factor ▨ 305
Fall incident ☐ 306

2

201c

201d

200

FIG.4

300

Bed exit □ ～301
Bed edge ▨ ～302
Torso up ■ ～303
Restless ▨ ～304

300

Risk Factor ▨ ～305
Fall incident □ ～306

2

201d

201c

200

FIG.5A

300

Bed exit ▨ ～301
Bed edge ■ ～302
Torso up ▨ ～303
Restless ■ ～304

300

Risk Factor ▨ ～305
Fall incident □ ～306

2

201d

201c

200

FIG.5B

Day

FIG.6A

Night

FIG.6B

300

Bed exit ☐—301
Bed edge ☐—302
Torso up ▨—303
Restless ☐—304

300

Risk Factor ☐—305
Fall incident ☐—306

2

201c

200

FIG.7

300

Bed exit ■—301
Bed edge ■—302
Torso up ▨—303
Restless ▨—304

300

Risk Factor ▨—305
Fall incident ☐—306

2

201c

200

FIG.8

300

Bed exit ■ ～301
Bed edge ■ ～302
Torso up ▦ ～303
Restless ▦ ～304

300

Risk Factor ▦ ～305
Fall incident □ ～306

2

201c

200

## FIG.9

300

Bed exit ■ ～301
Bed edge ■ ～302
Torso up ▦ ～303
Restless ■ ～304

300

Risk Factor ▦ ～305
Fall incident ■ ～306

201c
402
2

200

## FIG.10

FIG.11

FIG.12

FIG.13

FIG.14

FIG.15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013150523 A